(19) Europäisches Patentamt
European Patent Office
Office européen des brevets

(11) Veröffentlichungsnummer: **0 011 288**
**B1**

(12) # EUROPÄISCHE PATENTSCHRIFT

(45) Veröffentlichungstag der Patentschrift: **20.01.82**

(21) Anmeldenummer: **79104497.7**

(22) Anmeldetag: **14.11.79**

(51) Int. Cl.³: **C 07 C 21/24,** C 07 C 25/13, C 07 C 25/02, C 07 C 63/04, C 07 C 17/14, C 07 C 51/093

(54) **p-tert.-Butylbenzotribromid und deren am Kern durch Halogen substituierte Derivate, Verfahren zu ihrer Herstellung sowie ihrer Verwendung.**

(30) Priorität: **16.11.78 DE 2849663**

(43) Veröffentlichungstag der Anmeldung: **28.05.80 Patentblatt 80/11**

(45) Bekanntmachung des Hinweises auf die Patenterteilung: **20.01.82 Patentblatt 82/3**

(84) Benannte Vertragsstaaten: **AT BE CH DE FR GB IT NL**

(56) Entgegenhaltungen:
**DE-A-1 468 852**
**DE-C-478 084**

(73) Patentinhaber: **HOECHST AKTIENGESELLSCHAFT, Postfach 80 03 20, D-6230 Frankfurt/Main 80 (DE)**

(72) Erfinder: **Landauer, Franz, Dr., Liederbacher Strasse 7, D-6230 Frankfurt am Main 80 (DE)**
Erfinder: **Schaeffer, Georg, Dr., Herderstrasse 58, D-6238 Hofheim am Taunus (DE)**

EP 0 011 288 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# p-tert.-Butylbenzotribromid und deren am Kern durch Halogen substituierte Derivate, Verfahren zu ihrer Herstellung sowie ihrer Verwendung

p-tert.-Butylbenzotribromid und p-tert.-Butylbenzoylbromid und deren am Kern durch Halogen substituierte Derivate sind wertvolle neue Zwischenprodukte für die Herstellung der — gegebenenfalls am Kern durch Halogen substituierten — p-tert.-Butylbenzoesäure und deren Derivaten. Die Ester besitzen teilweise Krampflösende, teilweise auch fungizide Wirkung und sind daher von Bedeutung auf dem Pharma-Sektor. Bisher wurde die p-tert.-Butylbenzoesäure hauptsächlich hergestellt nach Grignard aus p-tert.-Butylphenylmagnesiumbromid und $CO_2$, oder durch Erhitzen von p-tert.-Butylbenzylbromid oder von 1-Isopropyl-4-tert.-butylbenzol mit Salpetersäure (s. Beilstein E III 9, System Nr. 944/H 560, S. 2525/26). Diese Verfahren weisen aber insbesondere für die technische Durchführung Nachteile auf. So ist die Grignard-Synthese für technische Prozesse sehr aufwendig und bei der Oxidation mit Salpetersäure sind ebenfalls aufwendige Anlagen zur Reinhaltung von Abwasser und Luft erforderlich.

Die Anwendung einer weiteren, für die Herstellung aromatischer Säuren bekannten Methode — nämlich die Verseifung von Benzotrichloriden mit Wasser — auf die Herstellung der p-tert.-Butylbenzoesäure erwiesen sich, wie eigene Versuche zeigten, als wenig vorteilhaft, weil bei der Herstellung des hier erforderlichen p-tert.-Butylbenzotrichlorids durch radikalische Chlorierung von p-tert.-Butyltoluol Produkte erhalten werden, deren organisch gebundenes Cl nur zum Teil unter den üblichen Bedingungen durch alkalische Hydrolyse wieder abgespalten werden kann (vgl. Houben-Weyl, «Methoden der organischen Chemie», Band II, S. 233, Stuttgart 1953), was auf eine nicht unbeträchtliche Kernchlorierung hindeutet. Dies ist — wenn man am Ende reine p-tert.-Butylbenzoesäure oder deren entsprechende Derivate haben will — natürlich ziemlich unerwünscht. In manchen Fällen werden zwar für die Herstellung von Pharmazeutika etc. auch am Kern durch Chlor substituierte Derivate der p-tert.-Butylbenzoesäure benötigt; bei der Chlorierung des p-tert.-Butyltoluols geht jedoch das Chlor oft gerade in eine solche Kernstellung, wo keine Chlorsubstitution gewünscht wird. Häufig wird auch überhaupt keine Kernsubstitution durch Chlor gewünscht, z.B. wenn der Kern bereits durch anderes Halogen (F, Br, J) substituiert ist und nicht noch durch Chlor weiter substituiert werden soll.

In dem Bestreben, auf einem günstigeren und wirtschaftlicheren Weg zur p-tert.-Butylbenzoesäure und insbesondere eines Säurehalogenids und deren am Kern durch Halogen substituierten Derivaten zu gelangen, wurde nun gefunden, dass dies über das neue Zwischenprodukt p-tert.-Butylbenzotribromid sowie dessen am Kern durch Halogen substituierte Derivate möglich ist.

Erfindungsgegenstand ist daher zunächst p-tert.-Butylbenzotribromid und dessen am Kern durch Halogen substituierte Derivate der nachstehenden Formel I:

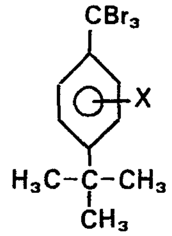
(I)

sowie dessen Verwendung zur Herstellung von p-tert.-Butylbenzoylbromid und dessen am Kern durch Halogen substituierte Derivate der nachstehenden Formel II:

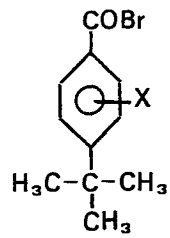
(II)

In beiden Formeln bedeutet X = H, F, Cl, Br oder J, vorzugsweise H, F, Cl, Br, insbesondere H.

Die unter Formel I fallenden Verbindungen sind also:

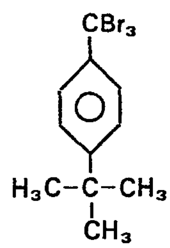
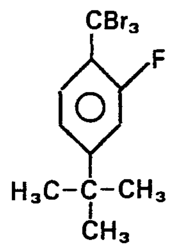
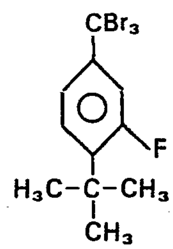

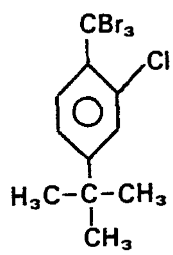
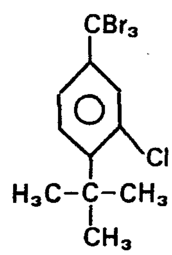
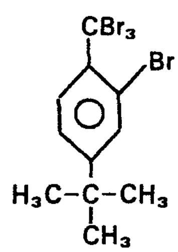

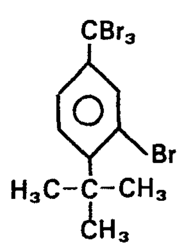
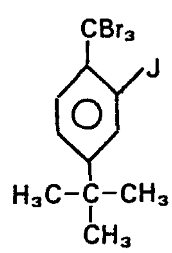
und
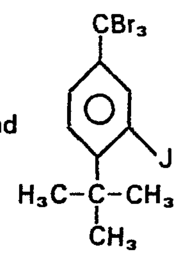

Unter die Formel II fallen:

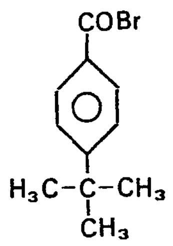
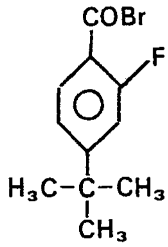
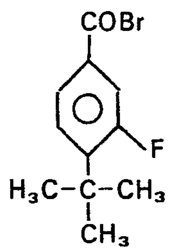
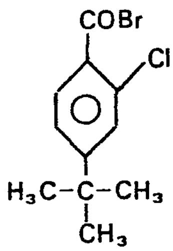
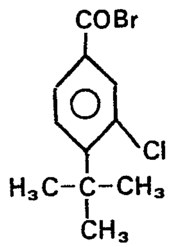
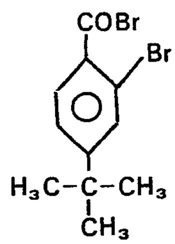
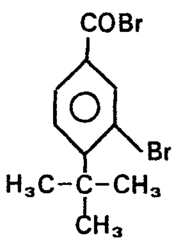
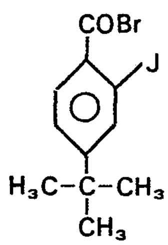

und

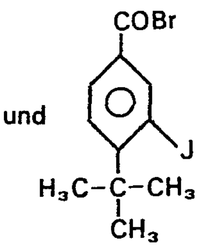

Erfindungsgegenstand ist weiterhin ein Verfahren zur Herstellung von p-tert.-Butylbenzotribromid sowie von dessen am Kern durch Halogen substituierten Derivaten und deren Verseifung zum p-tert.-Butylbenzoylbromid sowie von dessen am Kern druch Halogen substituierten Derivaten, das dadurch gekennzeichnet ist, dass man
a) p-tert.-Butyltoluol und dessen am Kern durch Halogen substituierte Derivate mit etwa 3 mol Brom/mol Kohlenwasserstoff bei Temperaturen von etwa 40-200°C, vorzugsweise etwa 80-160°C, gegebenenfalls unter der Einwirkung energiereicher Strahlung oder in Gegenwart von Radikalbildnern umsetzt, und
b) das dabei gebildete p-tert.-Butylbenzotribromid und dessen am Kern durch Halogen substituierte Derivate gegebenenfalls nach deren Isolierung bei erhöhter Temperatur mit etwa 1 mol Wasser/mol Tribromid, gegebenenfalls in Gegenwart von üblichen Verseifungskatalysatoren, zu p-tert.-Butylbenzoylbromid und dessen am Kern durch Halogen substituierten Derivaten verseift.

Ausgangsprodukte für das Verfahren sind das am Kern nicht substituierte p-tert.-Butyltoluol sowie dessen am Kern durch ein oder mehrere Halogenatome (F, Cl, Br, J) substituierte Derivate, wobei dann natürlich am Ende der Reaktion jeweils die gleiche Substitution des Kerns im Endprodukt vorhanden ist. Bevorzugte Ausgangsmaterialien sind — ausser dem am Kern unsubstituierten p-tert.-Butyltoluol — dessen am Kern nur durch 1 Halogenatom substituierte Derivate, also:

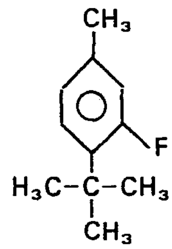
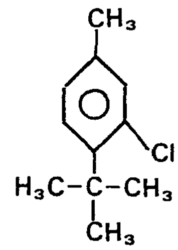
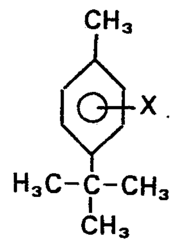
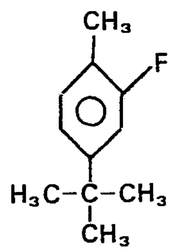
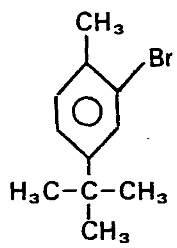
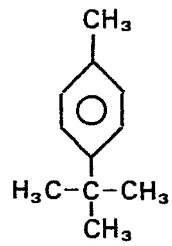
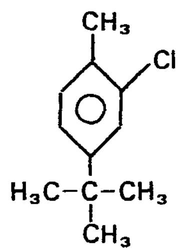
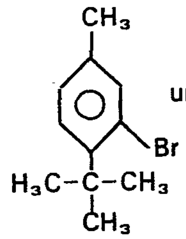

und

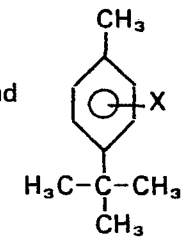

wobei von den substituierten Derivaten die F-, Cl- und Br-substituierten bevorzugt sind. Ausgehend von dem am Kern unsubstituierten p-tert.-Butyltoluol sowie dessen am Kern monohalogensubstituierten Derivaten werden nach dem erfindungsgemässen Verfahren dann die Verbindungen der vorgenannten Formeln I und II erhalten.

Bei der Durchführung des Verfahrens wird das jeweilige Ausgangsprodukt mit etwa 3(2,8-3,2)mol Brom/mol Ausgangsmaterial bei den angegebenen Temperaturen — etwa 40-200°C, vorzugsweise etwa 80-160°C — gegebenenfalls unter der Einwirkung energiereicher Strahlung oder in Gegenwart von Radikalbildnern umgesetzt. Als energiereiche Strahlung kommt vorzugsweise UV-Licht in Frage.

Geeignete Radikalbildner sind die für Seitenketten-Chlorierungen üblichen organischen Peroxide, Azoisobutyronitril etc.

Die energiereiche Strahlung oder die Gegenwart von Radikalbildnern sind für das Gelingen der Reaktion zwar nicht unbedingt erforderlich, jedoch in erheblichem Mass reaktionsbeschleunigend und daher vorteilhaft.

Das Brom kann bei dieser Reaktion entweder flüssig zugetropft oder gasförmig (nach dem Verdampfen) eingeleitet werden, wobei im letzteren Fall auch ein Inertgas ($N_2$, Argon etc.) zugesetzt werden kann. Die Bromierung kann sowohl ohne, als auch in einem geeigneten Lösungsmittel durchgeführt werden; als Lösungsmittel kommen

inerte — insbesondere halogenierte — kohlenwasserstoffe, wie z.B. Tetrachlorkohlenwasserstoff oder o-Dichlorbenzol in Betracht. Weiterhin ist die Durchführung der Reaktion drucklos oder unter erhöhtem Druck sowie sowohl diskontinuierlich, als auch kontinuierlich in geeigneten Apparaturen möglich.

Das bei dieser Bromierung gebildete p-tert.-Butylbenzotribromid sowie — wenn die am Kern durch Halogen substituierten Derivate des p-tert.-Butyltoluols als Ausgangsstoffe verwendet wurden — die entsprechenden am Kern durch Halogen substituierten Derivate besitzen keine unerwünschten Bromsubstituenten am aromatischen Kern und sind neue Verbindungen.

Sie können für die Weiterverarbeitung zum entsprechenden Benzoylbromid entweder isoliert und gereinigt (z.B. durch Umkristallisation) oder ohne Isolierung verseift werden. Die Verseifung erfolgt nach den für die Verseifung von Benzotrihalogeniden zu den entsprechenden Benzoylhalogeniden üblichen Methoden bei erhöhter Temperatur — vorzugsweise bei etwa 80-200°C, insbesondere etwa 90-150°C — mit einer äquivalenten Menge Wasser (etwa 1 mol/mol Tribromid), gegebenenfalls in Gegenwart von üblichen Verseifungskatalysatoren, z.B. von Metallhalogeniden wie $FeCl_3$, $FeBr_3$, $ZnCl_2$ oder auch von $H_2SO_4$ und dergleichen. Bei Zugabe von mehr als der berechneten (äquivalenten) Menge an Wasser kann selbstverständlich auch gleich bis zur entsprechenden Benzoesäure durchverseift werden. Die Verseifung kann z.B. sowohl drucklos, als auch unter erhöhtem Druck durchgeführt werden. Es können auch inerte Lösungsmittel, wie Kohlenwasserstoffe oder Chlorkohlenwasserstoffe zugesetzt werden; ferner ist noch die Verwendung etwa von Emulgatoren möglich, die eine gute Vermischung der organischen Phase mit dem zugegebenen Wasser bewirken.

Zur Herstellung des Säurebromids aus dem entsprechenden Benzotribromid kann auch so verfahren werden, dass man das Tribromid mit einer anderen Carbonsäure in Gegenwart von z.B. katalytischen Mengen an $ZnCl_2$ bei erhöhter Temperatur umsetzt, wobei sich neben dem p-tert.-Butylbenzoylbromid dann noch das Säurebromid der anderen zugesetzten Carbonsäure bildet.

Die Absorption des beim erfindungsgemässen Verfahren sich bildenden Bromwasserstoffs erfolgt zweckmässig in einer üblichen Absorptionsanlage; er kann dann direkt für andere chemische Reaktionen verwendet oder verkauft werden.

Nach dem erfindungsgemässen Verfahren werden p-tert.-Benzotribromid und p-tert.-Butylbenzoylbromid sowie deren am Kern durch Halogen substituierte Derivate in ausgezeichneten Ausbeuten erhalten. Die Produkte besitzen — ausser den schon in den Ausgangsmaterialien vorhandenen Kernsubstituenten — keine unerwünschten Bromsubstituenten mehr. Diese Tatsache ist ausserordentlich überraschend, da Halogenierungen mit Chlor und Brom normalerweise immer gleichartig verlaufen und da aber durch Seitenkettenchlorierung von p-tert.-Butyltoluol auch eine beträchtliche Kernchlorierung nicht zu vermeiden ist. Im Gegensatz dazu findet bei der Bromierung des p-tert.-Butyltoluols (und den entsprechenden Substitutionsprodukten) keine oder so gut wie keine unerwünschte Kernhalogenierung und darüber hinaus auch keine Bromierung der tert.-Butylgruppe statt. Die hohe Selektivität und Ausbeute des Verfahrens sowie die Tatsache, dass — im Gegensatz zu etlichen Verfahren des Standes der Technik — keinerlei besondere Abwasseraufbereitung erforderlich ist, stellen einen erheblichen Fortschritt dar.

Die Erfindung wird nun anhand des folgenden Beispiels näher erläutert. Auf das (Erfindungsbeispiel) folgt dann ein Vergleichsbeispiel, bei welchem als Halogenierungsmittel für das p-tert.-Butyltoluol anstelle des Broms Chlor verwendet wurde.

*Beispiel:*

a) p-tert.-Butylbenzotribromid

In einem 1l-4-Halskolben mit Rührer, Thermometer, Topftrichter und einem Rückflusskühler, welcher mit einer (mit Wasser gefüllten) Absorptionsanlage für Bromwasserstof verbunden ist, werden zu 148 g p-tert.-Butyltoluol (1 mol) bei 110-120°C unter UV-Bestrahlung im Verlaufe von 6-7 h 480 g Brom (3 mol) eingetropft. Nachdem etwa die Hälfte bis 2/3 der Brommenge zugegeben sind, wird die Temperatur auf 145-155°C erhöht.

Nach der Zugabe der gesamten Brommenge wird noch 15-30 min nachgerührt. Anschliessend wird der restliche Bromwasserstoff mit Stickstoff ausgeblasen.

Rohausbeute: 378 g (= 98,2% d. Th.)

Das Produkt wurde aus Isopropanol umkristallisiert.

Sein Fp. betrug dann 118-119°C.

Analyse: Gesamtbrom: 62,6%

verseifbares Brom: 62,1%

(Bestimmung: Verseifung mit alkoholischer KOH und argentometrische Bestimmung des ionogenen Broms)

Berechnetes verseifbares Brom: 62,3%

An Bromwasserstoff wurden 236 g (= 2,92 mol) gefunden, d.i. 97,3% d. Th.

b) p-tert.-Butylbenzoylbromid

In einem Rührkolben, versehen mit Thermometer, Dosierpumpe und Rückflusskühler, an dem eine Adsorptionsanlage für Bromwasserstoff angeschlossen ist, werden zu 770 g p-tert.-Butylbenzotribromid (2 mol) bei 110-120°C eine Lösung von 0,8 g Eisen-III-chlorid in 4 g $H_2O$ innerhalb von 5 min mit einer kleinen Dosierpumpe eingegeben. Im Verlauf von 1,5 h werden dann 32 g Wasser gleichmässig zugepumpt.

Der freiwerdende Bromwasserstoff wird in Wasser aufgefangen.

Es wurden gefunden: 319 g, das sind 98,5% d. Th. an HBr.

Rohausbeute an p-tert.-Butylbenzoylbromid: 465 g = 96,5% d. Th.

Durch Vakuumdestillation wurde das Säurebromid gereinigt.

$Kp_{0,15}$ 96-98°C.

| Analyse: | ber. | gef. |
|---|---|---|
| C | 54,7% | 54,4% |
| H | 5,4% | 5,5% |
| verseift Br | 32,2% | 33,6% |

*Vergleichsbeispiel:*

In genau dergleichen Apparatur und unter den gleichen Bedingungen wie im Erfindungsbeispiel wurden 296 g p-tert.-Butyltoluol (2 mol) durch Einleiten von 284 g (ca. 4,1 mol) Chlor im Verlauf von 3,5 h chloriert. Nach den Ausblasen des Chlorwasserstoffs betrug die Rohausbeute 415 g; darin wurden gefunden:
Gesamtchlor: 31,5%
verseifbares Chlor: 20,85% (Bestimmung: Verseifung mit alkoholischer KOH und argentometrische Bestimmung des ionogenen Chlors);
daraus berechnet sich an nicht abspaltbaren (Kern-) Chlor: 10,20%.

Etwa 1/3 des gesamten organisch gebundenen Chlors ist also für die Verseifung — hier zum p-tert.-Butylbenzaldehyd — wertlos.

Das Vergleichsbeispiel wurde zwar nur mit der für die Herstellung der Benzalstufe ($-CHCl_2$) erforderlichen Chlormenge durchgeführt; dabei fand jedoch offensichtlich schon eine ganz erhebliche Kernchlorierung statt. Wenn die Chlorierung daher mit der für die Herstellung der Benzotrichloridstufe ($-CCl_3$) erforderlichen höheren Chlormenge durchgeführt wird, kann keine geringere Kernchlorierung stattfinden.

Mit Sulforylchlorid anstelle des Chlors in diesem Vergleichsbeispiel wurde ein sehr ähnliches Ergebnis erhalten.

**Patentansprüche für die Vertragsstaaten: BE, CH, DE, FR, GB, IT, NL**

1. p-tert.-Butylbenzotribromid und dessen am Kern durch Halogen substituierte Derivate der Formel I:

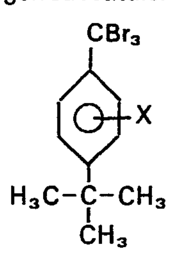

(I)

worin X = H, F, Cl, Br, J, vorzugsweise H, F, Cl, Br, insbesondere H.

2. Verfahren zur Herstellung von p-tert.-Butylbenzotribromid und dessen am Kern durch Halogen substituierten Derivaten, dadurch gekennzeichnet, dass man p-tert.-Butyltoluol und dessen am Kern durch Halogen substituierte Derivate mit etwa 3 mol Brom/mol oraganisches Ausgangsmaterial bei Temperaturen von etwa 40-200°C, vorzugsweise etwa 80-160°C, gegebenenfalls unter Einwirkung energiereicher Strahlung oder in Gegenwart von Radikalbildnern umsetzt.

3. Verfahren nach Anspruch 2, dadurch gekennzeichnet, dass man als am Kern durch Halogen substituierte Derivate des p-tert.-Butyltoluols die monosubstituierten Derivate der Formel III:

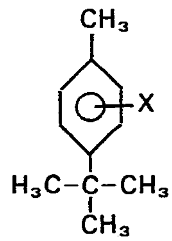

(III)

worin X = F, Cl, Br, J, insbesondere F, Cl oder Br, verwendet.

4. Verfahren nach Ansprüchen 2 und 3, dadurch gekennzeichnet, dass man als energiereiche Strahlung UV-Licht verwendet.

5. Verwendung von p-tert.-Butylbenzotribromid und dessen am Kern durch Halogen substituierten Derivate zur Herstellung von p-tert.-Butylbenzoylbromid und dessen am Kern durch Halogen substituierten Derivaten durch Verseifung mit etwa 1 mol Wasser/mol Tribromid bei erhöhter Temperatur, gegebenenfalls in Gegenwart von üblichen Verseifungskatalysatoren.

**Patentansprüche für den Vertragsstaat: AT**

1. Verfahren zur Herstellung von p-tert.-Butylbenzotribromid und dessen am Kern durch Halogen substituierten Derivaten, dadurch gekennzeichnet, dass man p-tert.-Butyltoluol und dessen am Kern durch Halogen substituierte Derivate mit etwa 3 mol Brom/mol organisches Ausgangsmaterial bei Temperaturen von etwa 40-200°C, vorzugsweise etwa 80-160°C, gegebenenfalls unter der Einwirkung energiereicher Strahlung oder in Gegenwart von Radikalbildnern umsetzt.

2. Verfahren nach Anspruch 1, dadurch gekennzeichnet, dass man als am Kern durch Halogen substituierte Derivate des p-tert.-Butyltoluols die monosubstituierten Derivate der Formel III:

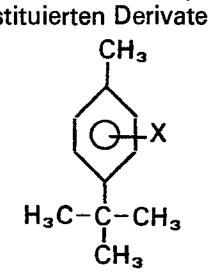

(III)

worin X = F, Cl, Br, J, insbesondere F, Cl oder Br, verwendet.

3. Verfahren nach Ansprüchen 1 und 2, dadurch gekennzeichnet, dass man als energiereiche Strahlung UV-Licht verwendet.

4. Verwendung von p-tert.-Butylbenzotribromid und dessen am Kern durch Halogen substituierten Derivate zur Herstellung von p-tert.-Butylbenzoylbromid und dessen am Kern durch Halogen substituierten Derivaten durch Verseifung mit etwa 1 mol Wasser/mol Tribromid bei

erhöhter Temperatur, gegebenenfalls in Gegenwart von üblichen Verseifungskatalysatoren.

**Claims for the Contracting States: BE, CH, DE, FR, GB; IT, NL**

1. p-tert.-Butylbenzotribromide and its derivatives substituted by halogen at the nucleus of the following formula I:

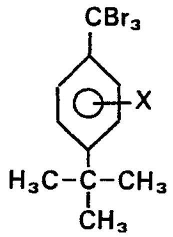

(I)

in which X is H, F, Cl, Br, I, preferably H, F, Cl, Br, and more preferably H.

2. Process for the manufacture of p-tert.-butylbenzotribromide and its derivatives substituted by halogen at the nucleus, characterized in reacting p-tert.-butyltoluene and its derivatives substituted at the nucleus by halogen with about 3 mol of bromine per mol of organic starting material, at a temperature of from about 40 to 200°C, preferably about 80 to 160°C, optionally under the action of high energy radiation or in the presence of radical-forming agents.

3. The process of claim 2, characterized in using as derivatives of p-ter.-butyltoluene substituted by halogen at the nucleus the monosubstituted derivatives of the formula III:

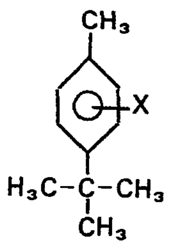

(III)

in which X is F, Cl, Br, I, preferably F, Cl, or Br.

4. The process of claims 2 and 3, characterized in using as high energy radiation ultraviolet light.

5. Using p-tert.-butylbenzotribromide and its derivatives substituted by halogen at the nucleus for the manufacture of p-tert.-butylbenzoylbromide and its derivatives substituted by halogen at the nucleus by saponification with about 1 mol of water for each mol of tribromide at elevated temperature, optionally in the presence of usual saponification catalysts.

**Claims for the Contracting State: AT**

1. Process for the manufacture of p-tert.-butylbenzotribromide and its derivatives substituted by halogen at the nucleus, characterized in reacting p-tert.-butyltoluene and its derivatives substituted at the nucleus by halogen with about 3 mol of bromine per mol of organic starting material, at a temperature of from about 40 to 200°C, preferably about 80 to 160°C, optionally under the action of high energy radiation or in the presence of radical-forming agents.

2. The process of claims 1, characterized in using as derivatives of p-tert.-butyltoluene substituted by halogen at the nucleus the monosubstituted derivatives of the formula III:

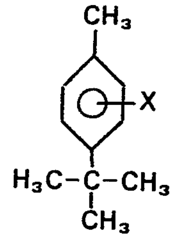

(III)

in which X is F, Cl, Br, I, preferably F, Cl, or Br.

3. The process of claims 1 and 2, characterized in using as high energy radiation ultraviolet light.

4. Using p-tert.-butylbenzotribromide and its derivatives substituted by halogen at the nucleus for the manufacture of p-tert.-butylbenzoylbromide and its derivatives substituted by halogen at the nucleus by saponification with about 1 mol of water for each mol of tribromide at elevated temperature, optionally in the presence of usual saponification catalysts.

**Revendications pour les Etats contractants: BE, CH, DE, FR, GB, IT, NL**

1. Tribromométhyl-1 tert.-butyl-4 benzène et ses dérivés halogénés sur le noyau, qui répondent à la formule I:

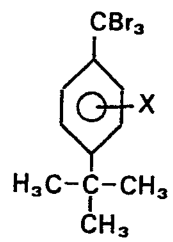

(I)

dans laquelle X représente H, F, Cl, Br ou I, de préférence H, F, Cl ou Br, plus spécialement H.

2. Procédé de préparation du tribromométhyl-1 tert.-butyl-4 benzène et de ses dérivés halogénés sur le noyau, caractérisé en ce qu'on fait réagir le p-tert.-butyltoluène ou ses dérivés halogénés sur le noyau avec environ 3 mol de brome par mole du corps de départ organique, à des températures d'environ 40 à 200°C, de préférence d'environ 80 à 160°C, éventuellement sous l'action d'un rayonnement de haute énergie ou en présence de générateurs de radicaux.

3. Procédé selon la revendication 2, caractérisé en ce qu'on utilise, comme dérivés du p-tert.-butyltoluène halogénés sur le noyau, les dérivés monosubstitués répondant à la formule III:

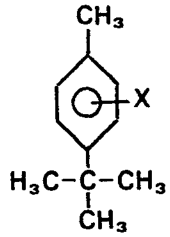

(III)

dans laquelle X représente F, Cl, Br ou I, plus particulièrement F, Cl ou Br.

4. Procédé selon l'une des revendications 2 ou 3, caractérisé en ce qu'on utilise une lumière ultraviolette comme rayonnement de haute énergie.

5. Application du tribromométhyl-1 tert.-butyl-4 benzène et de ses dérivés halogénés sur le noyau à la préparation du bromure de p-tert.-butylbenzoyle et de ses dérivés halogénés sur le noyau par saponification avec environ 1 mol d'eau par mole de tribromure, à température élevée, éventuellement en présence de catalyseurs de saponification usuels.

## Revendications pour l'Etat: AT

1. Procédé de préparation du tribromométhyl-1 tert.-butyl-4 benzène et de ses dérivés halogénés sur le noyau, caractérisé en ce qu'on fait réagir le p-tert.-butyltoluène ou ses dérivés halogénés sur le noyau avec environ 3 mol de brome par mole du corps de départ organique, à des températures d'environ 40 à 200°C, de préférence d'environ 80 à 160°C, éventuellement sous l'action d'un rayonnement de haute énergie ou en présence de générateurs de radicaux.

2. Procédé selon la revendication 1, caractérisé en ce qu'on utilise, comme dérivés du p-tert.-butyltoluène halogénés sur le noyau, les dérivés monosubstitués répondant à la formule III:

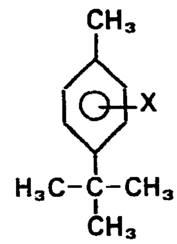

(III)

dans laquelle X représente F, Cl, Br ou I, plus particulièrement F, Cl ou Br.

3. Procédé selon l'une des revendications 1 ou 2, caractérisé en ce qu'on utilise une lumière ultraviolette comme rayonnement de haute énergie.

4. Utilisation du tribromométhyl-1 tert.-butyl-4 benzène et de ses dérivés halogénés sur le noyau pour la préparation du bromure de p-tert.-butylbenzoyle et de ses dérivés halogénés sur le noyau, par saponification avec environ 1 mol d'eau par mole du tribromure, à température élevée, éventuellement en présence de catalyseurs de saponification usuels.